# EUROPEAN PATENT APPLICATION

(11) **EP 4 218 931 A1**
(43) Date of publication of application: **02.08.2023**
(21) Application number: 23150743.5
(22) Date of filing: 09.01.2023
(51) Int. Cl.: A61P 35/00, A23L 29/00, A23L 33/125

(54) **DIETARY SUPPLEMENT FOR ANTINEOPLASTIC THERAPIES AND TREATMENT OF LOWER ABDOMINAL PAIN**

(30) Priority: 26.01.2022 IT 202200001274
(71) Applicant: Geophyt S.r.L., 15067 Novi Ligure (AL) (IT)
(72) Inventor: MERLO, Enrico, I-15067 NOVI LIGURE (Alessandria) (IT)
(74) Representative: Lunati & Mazzoni S.r.L.

(57) **Abstract**

It is provided a dietary supplement for antineoplastic therapies and treatment of lower abdominal pain comprising Klamath algae, probiotics and oligosaccharide fruit present in an amount of 52% to 62% by weight of said supplement.

## Description

It is an object of the present invention to provide a dietary supplement for antineoplastic therapies and treatment of lower abdominal pain, such as radiotherapy and/or chemotherapy, of the type specified in the preamble to the first claim.

In particular, the present invention has as its object a dietary supplement capable of improving the efficacy of both radiotherapy and cancer therapies, as well as relieving the pain from which the lower abdomen can commonly be affected.

In Italy, as in the rest of the world, the incidence of malignant tumours is constantly increasing, with around 400,000 new cases in 2012, representing the second leading cause of death, after cardiovascular diseases, and 30% of all deaths.

The non-surgical therapies used to treat the malignant tumours, such as chemotherapy, radiotherapy or a combination of both, are increasingly widespread and effective.

In the case of the radiotherapy, its effectiveness is often affected by the formation of air in the user's rectum as CT scanning of the rectal area is provided. The air in the rectum can essentially cause displacement of the organs above it, such as the prostate and endometrium, and therefore its formation must always be controlled.

Normally, although the patients follow diets to prevent the formation of air, it is almost always present and it is therefore necessary to carry out the unpleasant air drains before the radiation therapy, taking care not to expel urine.

As far as chemotherapy is concerned, on the other hand, it is important that the area destroyed by cancer treatments is treated to re-establish the normal intestinal flora. Therefore, the organisms that rapidly colonise and replicate in the treated area must also be introduced into the rectal zone.

Moreover, as is well known, the human rectal area can be affected by pain resulting from various factors, e.g. irritable bowel syndrome 'IBS', diverticulosis, prostatitis, proctitis, haemorrhoids, and even more.

In order to ensure adequate cancer therapy and treatment of the above-mentioned pains, numerous food supplements or drugs have been produced.

The known technique described includes some major drawbacks.

In particular, none of the known products has a relevant efficacy for all the problems described above. Therefore, the patient who has to follow oncological therapies and is at the same time suffering from the above-mentioned pathologies involving pain, must often necessarily resort to a plurality of different supplements and/or drugs.

These, however, must be compatible with each other and it is very important to avoid side effects of the one being able to affect, if not amplify, the side effects of the others. It must also be considered that the efficacy of some supplements and drugs may also be nullified by the presence of other actives and, therefore, it may also be impossible to handle all problems in the same time period.

In this situation, the technical task underlying the present invention is to devise a dietary supplement for antineoplastic therapies and treatment of lower abdominal pain capable of substantially obviating at least some of the aforementioned drawbacks.

In the context of said technical task, it is an important aim of the invention to obtain a dietary supplement for antineoplastic therapies and treatment of lower abdominal pain which enables to increase the effectiveness of radiotherapy and oncological therapies and, at the same time, to prevent and alleviate the pain which may be present in the lower abdomen.

Therefore, another important purpose of the invention is to make a dietary supplement for antineoplastic therapies and treatment of lower abdominal pain that can be freely used by any user.

The specified technical task and purposes are achieved by a dietary supplement for antineoplastic therapies and treatment of lower abdominal pain as claimed in the appended claim 1.

Preferred technical solutions are disclosed in the dependent claims.

In the present document, the measurements, values, shapes and geometric references (such as perpendicularity and parallelism), when associated with words like "about" or other similar terms such as "approximately" or "substantially", are to be considered as except for measurement errors or inaccuracies due to production and/or manufacturing errors, and, above all, except for a slight divergence from the value, measurements, shape, or geometric reference with which it is associated. For instance, these terms, if associated with a value, preferably indicate a divergence of not more than 10% of the value.

Moreover, when used, terms such as "first", "second", "higher", "lower", "main" and "secondary" do not necessarily identify an order, a priority of relationship or a relative position, but can simply be used to clearly distinguish between their different components.

Unless otherwise specified, as results in the following discussions, terms such as "treatment", "computing", "determination", "calculation", or similar, refer to the action and/or processes of a computer or similar electronic calculation device that manipulates and/or transforms data represented as physical, such as electronic quantities of registers of a computer system and/or memories in, other data similarly represented as physical quantities within computer systems, registers or other storage, transmission or information displaying devices.

The measurements and data reported in this text are to be considered, unless otherwise indicated, as performed in the International Standard Atmosphere ICAO (ISO 2533:1975).

The dietary supplement for antineoplastic therapies and treatment of lower abdominal pain is essentially intended to allow the reduction, if not complete elimination, of air in the rectum while allowing bacterial colonies to flourish within the rectum to re-establish the intestinal flora and allow any lower abdominal pain to be relieved.

To this end, the dietary supplement includes Klamath algae, probiotics and at least one oligosaccharide fruit, also known as FOS.

The Klamath algae consists of a group of algae, generally green or blue in colour, belonging to the species Aphanizomenon flos-aquae. The name originates from the fact that this algae grows and is harvested from Klamath Lake located in the United States and, more precisely, in Oregon.

The algae are generally harvested during warm periods and are then accumulated inside tanks and subjected to a heat treatment that brings them to temperatures close to 0°.

Following micro-filtration, they are purified and freeze-dried for marketing.

The dietary supplement preferably includes Klamath algae in powder form. In addition, the supplement may include pure Klamath algae or an extract of it.

An example of Klamath algae is that marketed by Geophyt S.r.l. under the trademark Proklama^{™}, or that marketed by Nutrigea under the trademark Klamin^{™}.

In particular, the Klamath algae is preferably present in a dose by weight of between 6% and 16% of the total weight of the supplement.

More specifically, the Klamath algae may be present in a dose by weight of between 9% and 13% of the total weight of the supplement.

Appropriately, the Klamath algae may be present in a dose by weight of 11% of the total weight of the supplement, e.g. 11.36%.

Naturally, now as later, when a percentage dose is given, the percentage is understood to be rounded. Thus, a percentage of 11% includes all percentages strictly between 10.5% and 11.5%.

The probiotics, as is well known, are essentially live micro-organisms that, when administered in adequate quantities, benefit the health of the host organism.

Among the probiotics, the bacteria or milk enzymes are particularly popular.

The supplement probiotics preferably include lactic ferments. In particular, probiotics comprise one or more milk enzymes from a choice of Lactobacillus Acidophilus, Lactobacillus Casei, Lactobacillus Rhamnosus, Bifidobacterium Longum and Lactobacillus Salivarius.

Specifically, the probiotics are present in 27% to 37% by weight of the amount by weight of said supplement.

More specifically, probiotics may be present in a dose by weight of between 30% and 34% relative to the total weight of the supplement.

Appropriately, the Klamath algae may be present in a dose by weight of 32% relative to the total weight of the supplement, e.g. 31.82%.

In addition, the probiotics can include all the milk enzymes listed above.

In this case, preferably Lactobacillus Acidophilus is 26% to 32% by weight of the probiotics.

More specifically, the Lactobacillus Acidophilus may be in a dose by weight of between 28% and 30% relative to the amount by weight of the probiotics.

Appropriately, the Lactobacillus Acidophilus may be 29% by weight of the amount by weight of probiotics, e.g. 9.09%.

In addition, the Bifidobacterium Longum, the Lactobacillus Casei, the Lactobacillus Rhamnosus and the Lactobacillus Salivarius may each be present in a dose by weight of between 15% and 21% relative to the amount by weight of probiotics.

More specifically, the Bifidobacterium Longum, the Lactobacillus Casei, the Lactobacillus Rhamnosus and the Lactobacillus Salivarius may each be present in a dose by weight of between 17% and 19% relative to the amount by weight of probiotics. Appropriately, the Bifidobacterium Longum, the Lactobacillus Casei, the Lactobacillus Rhamnosus and the Lactobacillus Salivarius may each be 18% by weight of the amount by weight of the probiotics, e.g. 17.86%.

In addition, each of the lactic ferments can define a concentration of colony-forming units, also known as c.f.u., between 80 mld/g and 120 mld/g. More specifically, the concentration can be as high as 100 mld/g.

The oligosaccharide fruit is, advantageously, present in doses of between 52% and 62% by weight of the supplement.

More specifically, the oligosaccharide fruit may be present in a dose by weight of between 56% and 58% relative to the amount by weight of the supplement. Appropriately, the oligosaccharide fruit may be present in a dose by weight of 57% relative to the amount by weight of the supplement, e.g. 56.82%.

Each of the components of the supplement may be in granular or powder form. Therefore, overall, the supplement may also be in granular form.

Furthermore, in the preferred, but not exclusive, embodiment form of the invention the supplement preferably comprises, or consists of, Klamath algae in a dose by weight of 11% relative to the amount by weight of the supplement, said oligosaccharide fruit in a dose by weight of 57% relative to the amount by weight of the supplement, Lactobacillus Acidophilus in a dose by weight of 9% relative to the amount by weight of the supplement, Bifidobacterium Longum 6% by weight of the amount by weight of the supplement, Lactobacillus Casei 6% by weight of the amount by weight of the supplement, Lactobacillus Rhamnosus 6% by weight of the amount by weight of the supplement, and Lactobacillus Salivarius 6% by weight of the amount by weight of the supplement.

The latter may be included in sachets or stick packs.

For example, one dose of the dietary supplement may be about 880 mg in total.

Thus, the supplement may comprise, or consist of, Klamath algae in an amount of 100 mg, oligosaccharide fruit in an amount of 500 mg, Lactobacillus Acidophilus in an amount of 80 mg, Bifidobacterium Longum in an amount of 50 mg, Lactobacillus Casei in an amount of 50 mg, Lactobacillus Rhamnosus in an amount of 50 mg, and Lactobacillus Salivarius in an amount of 50 mg.

The dietary supplement for antineoplastic therapies and treatment of lower abdominal pain according to the invention achieves important advantages.

In fact, the dietary supplement simultaneously
- induce the development of 280 billion colony-forming units in the human intestine,
- reduce meteorism,
- reduce percentage movement of the volume of the rectum (PVCR),
- prevent and reduce intestinal toxicity from radiotherapy,
- prevent and reduce genitourinary toxicity from radiotherapy,
- decrease abdominal pain,
- prevent and reduce diarrhoea
- reduce or eliminate abdominal tension, and
- prevent and decrease the occurrence of prostatitis or proctitis.

Therefore, the supplement is suitable for increasing the efficacy of radiotherapy and cancer therapies, while at the same time soothing pain that may be present in the lower abdomen.

In addition, the supplement combines in a single product all the features necessary to perform the aforementioned functions and therefore does not necessarily have to be combined with other supplements or drugs.

In any event, the dietary supplement according to the invention can substantially interact with almost all other supplements or drugs and is free of lactose, GMO, gluten and iodine.

Therefore, the supplement is substantially suitable for use by any patient.

The invention is susceptible to variations within the scope of the inventive concept as defined by the claims.

Within that scope, all details are substitutable by equivalent elements and the materials, shapes and dimensions can be any.

## Claims

1. Dietary supplement for antineoplastic therapies and treatment of lower abdominal pain comprising Klamath algae, probiotics and **characterised by** comprising oligosaccharide fruit present in a dose by weight of between 52% and 62% in relation to the amount by weight of the said supplement.

2. Supplement according to claim 1, wherein said oligosaccharide fruit is present in a dose by weight of between 56% and 58% relative to the amount by weight of said supplement.

3. Supplement according to claim 1, wherein said Klamath alga is present in a dose by weight of between 6% and 16% relative to the amount by weight of said supplement.

4. Supplement according to any one of the preceding claims, wherein said probiotics are present in a dose by weight of between 27% and 37% relative to the amount by weight of said supplement.

5. Supplement according to at least one preceding claim, wherein said probiotics comprise one or more lactic acid bacteria from a choice of Lactobacillus Acidophilus, Lactobacillus Casei, Lactobacillus Rhamnosus, Bifidobacterium Longum and Lactobacillus Salivarius.

6. Supplement according to any of the preceding claims, wherein said Lactobacillus Acidophilus is in a dosage by weight of between 26% and 32% relative to the amount by weight of said probiotics and said Bifidobacterium Longum, said Lactobacillus Casei, said Lactobacillus Rhamnosus and said Lactobacillus Salivarius are each in a dosage by weight of between 15% and 21% relative to the amount by weight of said probiotics.

7. Supplement according to any preceding claim, wherein said lactic acid bacteria each define a concentration of colony forming units between 80 bn/g and 120 bn/g.

8. Supplement according to any preceding claim, in granular form.

9. Supplement according to any preceding claim, comprising:
- said Klamath algae in a proportion by weight of 11% of the amount by weight of said supplement,
- said oligosaccharide fruit in a dose by weight of 57% relative to the amount by weight of said supplement,
- said Lactobacillus Acidophilus in a dose by weight of 9% by weight in relation to the quantity by weight of the said supplement,
- said Bifidobacterium Longum in a dose by weight equal to 6% of the quantity by weight of the said supplement,
- said Lactobacillus Casei in a dose by weight equal to 6% of the quantity by weight of the said supplement,
- said Lactobacillus Rhamnosus in a dose equal to 6% by weight of the amount by weight of said supplement, and
- said Lactobacillus Salivarius in a dose equal to 6% by weight of the amount by weight of that supplement.

10. Supplement according to the preceding claim, comprising:
- said Klamath algae in an amount equal to 100 g,
- said oligosaccharide fruit in a quantity of 500 g,
- said Lactobacillus Acidophilus in a quantity of 80 g, and
- said Bifidobacterium Longum in a quantity of 50 g,
- said Lactobacillus Casei in a quantity of 50 g,
- said Lactobacillus Rhamnosus in a quantity of 50 g, and
- said Lactobacillus Salivarius in a quantity of 50 g.
